# EUROPEAN PATENT APPLICATION

(11) **EP 0 530 166 A2**
(43) Date of publication of application: **03.03.1993**
(21) Application number: 92850198.0
(22) Date of filing: 24.08.1992
(51) Int. Cl.: C07C 229/64, C07C 229/56, C07C 323/63, A61K 31/195

(54) **3-Hydroxy anthranilic acid derivatives for inhibiting 3-hydroxy anthranilate oxygenase**

(30) Priority: 29.08.1991 US 752088
(71) Applicant: Astra Aktiebolag, 151 85 Södertälje (SE); THE UNIVERSITY OF MARYLAND AT BALTIMORE, Baltimore Maryland 21203 (US)
(72) Inventor: Björk, Susanna Karin Maria, S-151 59 Södertälje (SE); Carpenter, Barry K., Slaterville Springs, New York 14881-9401 (US); Gotthammar, Kristina Birgitta, S-132 31 Saltsjö-Boo (SE); Hagberg, Curt-Eric, S-194 51 Upplands-Väsby (SE); Högberg, Thomas, S-232 51 Akarp (SE); Linderberg, Mats Torbjörn, S-151 54 Södertälje (SE); Schwarcz, Robert, Baltimore, Maryland 21209 (US); Stening, Göran Bertil, S-151 51 Södertälje (SE)
(74) Representative: Hjertman, Ivan T.

(57) **Abstract**

The present invention relates to novel derivatives of 3-hydroxyanthranilic acid of the general formula I, methods for their preparation, novel pharmaceutical compositions and the use thereof for inhibiting the emzyne 3-hydroxyanthranilate oxygenase, 3-HAO, responsible for the production of the endogenous neurotoxin quinolinic acid, QUIN.

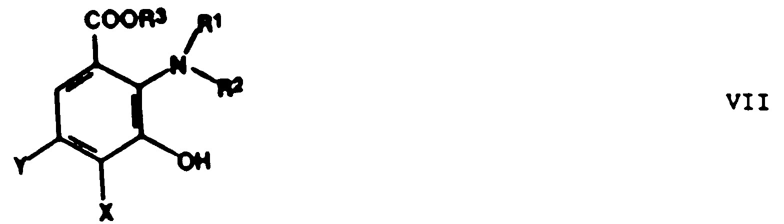

## Description

### Field of the invention

The present invention relates to novel derivatives of 3-hydroxyanthranilic acid, 3-HANA, methods for their preparation, novel pharmaceutical compositions and the use thereof for inhibiting the enzyme 3-hydroxyanthranilate oxygenase, 3-HAO, responsible for the production of the endogenous neurotoxin quinolinic acid, QUIN.

### Background of the invention

3-HAO is the enzyme in the catabolic pathway of tryptophan responsible for the conversion of 3-hydroxyanthranilic acid into quinolinic acid. Both QUIN and its biosynthetic enzyme 3-HAO have been identified in rodent as well as in human brain tissue. QUIN is a potent exitoxin acting through the N-methyl-D-aspartate (NMDA) receptor and has recently gained attention for its putative role as a pathogen in neurodegenerative disorders, such as Huntington's disease, stroke/cerebral ischemia, hypoxia, Alzheimer's disease and the AIDS dementia complex. Inhibitors of 3-HAO activity are of obvious therapeutic interest in diseases which can be traced to an overabundance of quinolinic acid.

### Prior art

4-halogenated substrate analogs have been described as inhibitors of 3-HAO activity. In 1980 it was shown by Parli CJ, Krieter P and Schmedt B, in "Metabolism of 6-chlorotryptophan to 4-chloro-3-hydroxyanthranilic acid: A potent inhibitor of 3-hydroxyanthranilic acid oxidase", Arch Biochem and Biophys 203, pp 161-166, 1980, that 4-chloro-3-hydroxyanthranilic acid, a metabolite of 6-chlorotryptophan, is a potent inhibitor of 3-HAO in rat and guinea pig liver and kidney. Later it was verified by Heyes MP, Hutto B and Markey SP, in "4-Chloro-3-hydroxy-anthranilate inhibits brain 3-hydroxyanthranilate oxidase", Neurochem Int 13, pp 405-408, 1988, that 4-chloro-3-hydroxyanthranilic acid also is an inhibitor of rat brain 3-HAO. In 1989 Todd WP, Carpenter BK and Schwarcz R, in "Preparation of 4-halo-3-hydroxyanthranilates and demonstration of their inhibition of 3-hydroxyanthranilate oxygenase activity in rat and human brain tissue", Prep Biochem 19, pp 155-165, 1989, showed that 4-fluoro-, 4-chloro- and 4-bromo-3-hydroxyanthranilic acid had very similar blocking potencies of 3-HAO in rat as well as in human brain.

### Brief description of the invention

The present invention relates to compounds able to inhibit the enzyme 3-HAO with IC₅₀ values superior to compounds according to the prior art.

The present invention, thus, is related to a compound of the general formula I 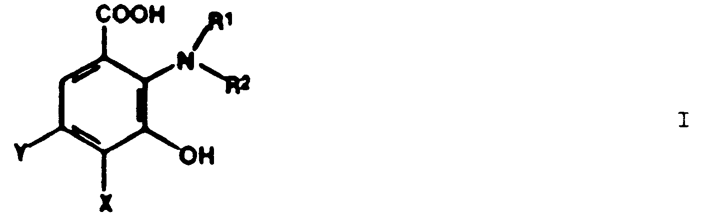
wherein R¹ and R² are the same or different and selected from H and alkyl; and X and Y are the same or different and selected from alkoxy, alkyl, alkylthio, fluoroalkyl, halogen, hydroxy, nitrile, OCF₃ and SCF₃, or a pharmaceutically acceptable salt thereof.

Another object of the invention is a process for the preparation of the compound of formula I by
A) in case R¹ and R² are the same or different and selected from H and alkyl, Y is selected from Cl, Br and I, X is selected from alkoxy, alkyl, alkylthio, fluoralkyl, halogen, nitrile, OCF₃ and SCF_{3;}
   halogenating a compound of formula II 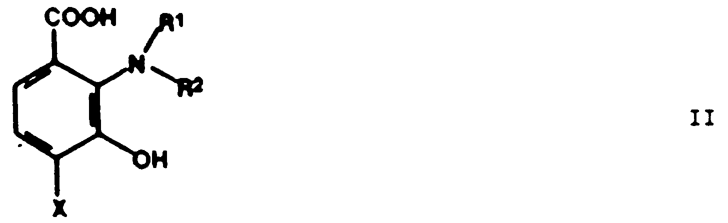 wherein R¹, R² and X are as defined in A) above, or
B) in case R¹ and R² are H, X and Y are the same or different and selected from alkoxy, alkyl, alkyltio, fluoroalkyl, halogen, hydroxy, nitrile, OCF₃ and SCF₃;
   deprotecting a compound of formula III 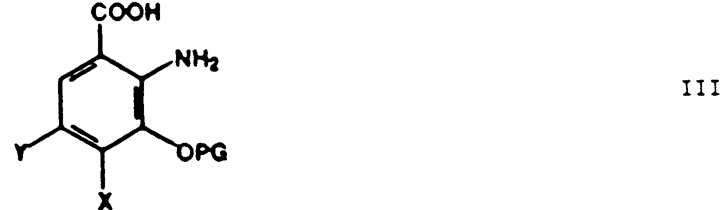 wherein R¹, R², X and Y are as defined in B) above and PG is a protecting group, or
C) in case R¹ and R² are H, X and Y are the same or different and selected from alkoxy, alkyl, alkylthio, fluoroalkyl, halogen, hydroxy, nitrile, OCF₃ and SCF₃;
   reducing a compound of formula IV 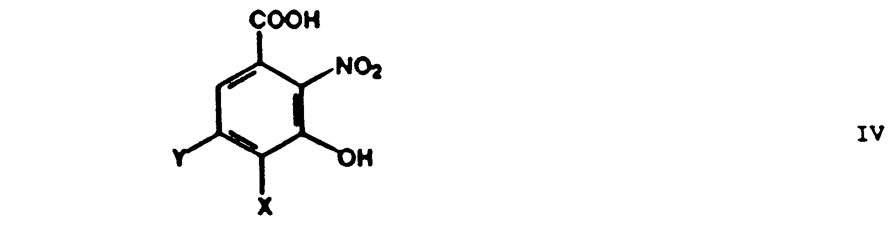 wherein X and Y are as defined in C) above, or
D) in case R¹ and R² are the same or different and selected from H and alkyl, X is Cl, Br, or I and Y is selected from alkoxy, alkyl, fluoroalkyl and halogen;
   halogenating a compound of formula V 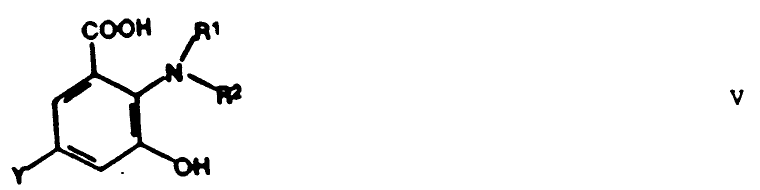 wherein R¹, R², and Y are as defined in D) above, or
E) in case R¹ is alkyl, R² is H or alkyl and X and Y are selected from alkoxy, alkyl, alkylthio, fluoroalkyl, halogen, hydroxy, nitrile, OCF₃ and SCF_{3;}
   alkylating a compound of formula VI 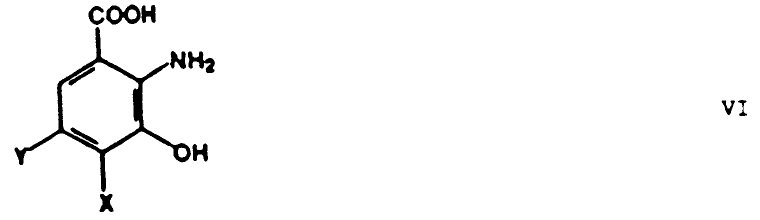 wherein X and Y are as defined in E) above, or
F) in case R¹ and R² are the same or different and selected from H and alkyl, X and Y are the same or different and selected from alkoxy, alkyl, alkylthio, fluoroalkyl, halogen, hydroxy, nitrile, OCF₃ and SCF₃;
   deesterifying a compound of formula VII

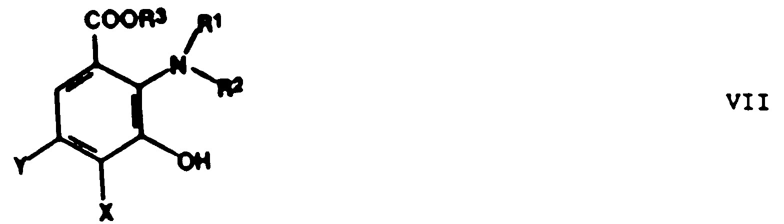

wherein R¹, R², X and Y are as defined in G) above and R³ is selected from alkyl, benzyl, 1,1,1-trichloroethyl and 2-(trimethylsilyl)ethoxymethyl (SEM) .

The present invention is also related to a pharmaceutical formulation containing a compound of formula I as active ingredient and a pharmaceutically acceptable carrier, the use of said compound for the manufacture of a medicament for the prevention or treatment of neurodegeneration.

Further objects of the invention are synthesis intermediates for the preparation of the compound of formula I, namely a compound of the general formula II 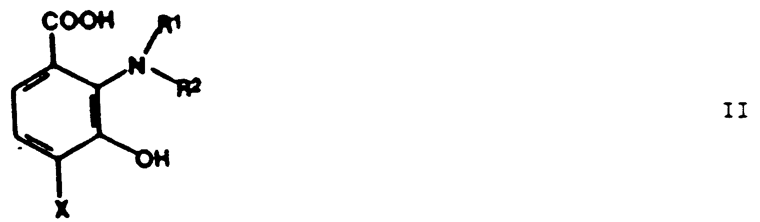
wherein R¹ and R² are the same or different and selected from H and alkyl, X is selected from alkoxy, alkyl, alkylthio, fluoralkyl, halogen, nitrile, OCF₃ and SCF₃, with the proviso that the compounds of formula II wherein R¹ and R² are H and X is OMe, OEt, Cl, Br or F are excluded,
a compound of the general formula III 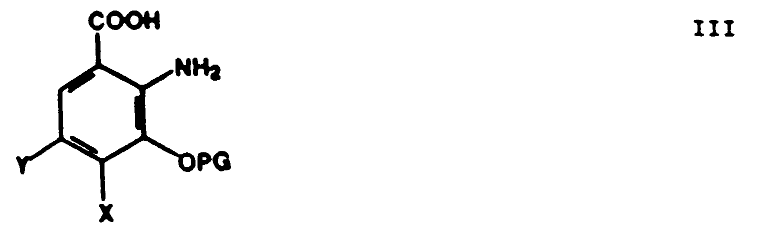
wherein X and Y are the same or different and selected from alkoxy, alkyl, alkylthio, fluoroalkyl, halogen, hydroxy, nitrile, OCF₃ and SCF₃ and PG is a protecting group, with the proviso that the compounds of formula III wherein PG is Me, X is OMe and Y is OMe; PG is Me, and X and Y are Cl; and PG is Me, X is OMe and Y is Cl are excluded,
a compound of the general formula IV 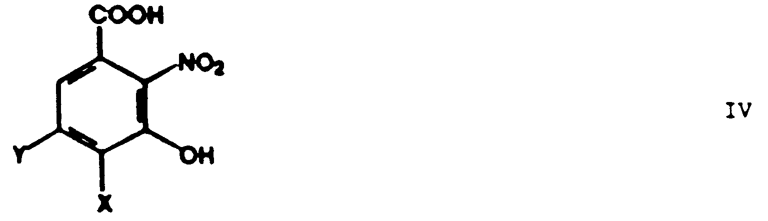
wherein X and Y are the same or different and selected from alkoxy, alkyl, alkylthio, fluoroalkyl, halogen, hydroxy, nitrile, OCF₃ and SCF₃,
a compound of the general formula V 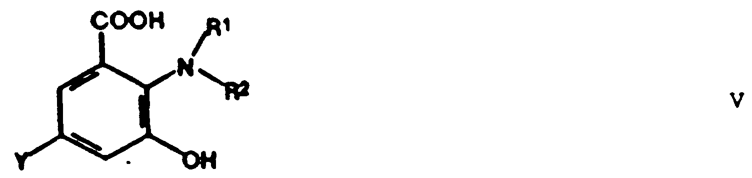
wherein R¹ and R² are the same or different and selected from H and alkyl, and Y is selected from alkoxy, alkyl, fluoroalkyl and halogen,
a compound of the general formula VI 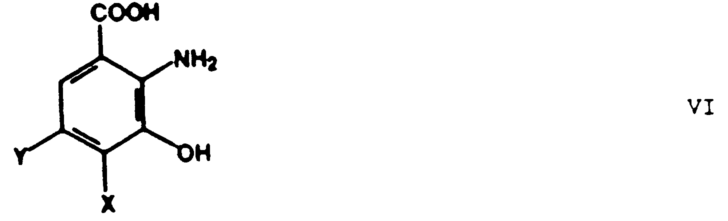
wherein X and Y are selected from alkoxy, alkyl, alkylthio, fluoroalkyl, halogen, hydroxy, nitrile, OCF₃ and SCF₃, and
a compound of the general formula VII 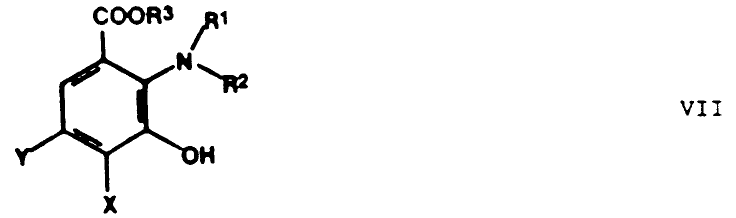
wherein R¹ and R² are the same or different and selected from H and alkyl, X and Y are the same or different and selected from alkoxy, alkyl, alkylthio, fluoroalkyl, halogen, hydroxy, nitrile, OCF₃ and SCF₃ and R³ is selected from alkyl, benzyl, 1,1,1-trichloroethyl and SEM.

### Detailed description of the invention

In the general formulas above "alkyl" may represent a straight or branched lower alkyl, preferably a C₁-C₆ alkyl. Examples of said lower alkyl include methyl, ethyl, n-propyl, iso-propyl, n-butyl, iso-butyl, sec-butyl, t-butyl and straight- and branched-chained pentyl and hexyl.

Furthermore, in the general formulas above "alkoxy" may represent a lower alkoxy, preferably a C₁-C₆ alkoxy. Examples of said lower alkoxy include methoxy, ethoxy, n-propoxy, iso-propoxy, n-butoxy, iso-butoxy, sec-butoxy, t-butoxy and straight- and branched-chain pentoxy and hexoxy.

"Halogen" may be selected from iodo, fluoro, chloro and bromo in the general formulas above.

Among the most preferred compounds of formula I according to the present invention are
5-bromo-4-chloro-3-hydroxyanthranilic acid,
4,5-dichloro-3-hydroxyanthranilic acid,>
4-bromo-5-ethoxy-3-hydroxyanthranilic acid,
4,5-dibromo-3-hydroxyanthranilic acid,
4-bromo-3-hydroxy-5-methylanthranilic acid,
4,5-dibromo-3-hydroxy-N-methyl-anthranilic acid, and
pharmaceutically acceptable salts thereof.

The best mode of carrying out the invention known at present is to use 5-bromo-4-chloro-3-hydroxyanthranilic acid, 4,5-dichloro-3-hydroxyanthranilic acid 4,5-dibromo-3-hydroxyanthranilic acid or 4-bromo-3-hydroxy-5-methylanthranilic acid.

The compounds according to the present invention may be used in connection with prevention or treatment of neurodegeneration, especially in connection with conditions such as stroke, cerebral ischaemia, hypoxia, epilepsy and in neurodegenerative diseases such as Alzheimer's disease, multi-infarct dementia, Huntington's disease and the AIDS dementia complex.

Below the methods for the preparation of the compound of formula I will be described in detail.

### Methods of preparation

The compounds of the formula 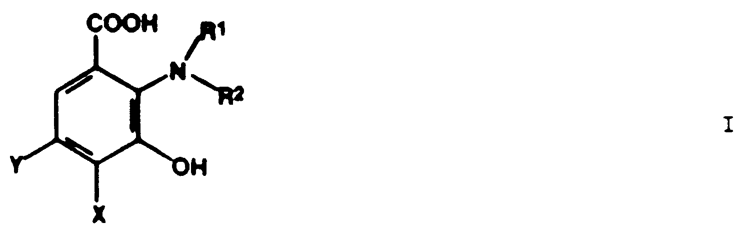
wherein R¹ and R² being the same or different and selected from H and alkyl, X and Y being the same or different and selected from alkoxy, alkyl, alkylthio, fluoroalkyl, halogen, hydroxy, nitrile, OCF₃, SCF₃, may be prepared by one of the following methods.

### Method A.

The compounds of formula I wherein R¹ and R² being the same or different and selected from H and alkyl, Y selected from Cl, Br and I, X selected from alkoxy, alkyl, alkylthio, fluoroalkyl, halogen, nitrile, OCF₃ and SCF₃ may be prepared from compounds of formula II 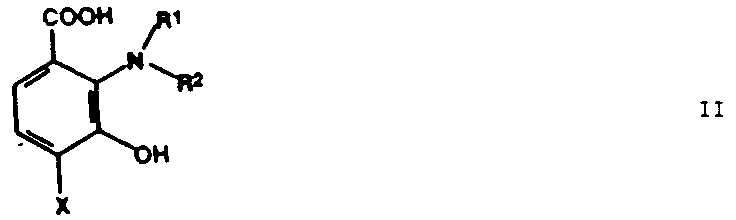
where R¹ , R², and X are as defined in formula I in method A, for example by halogenating with a solution of the elemental halogen in acetic acid or other suitable solvents between 0°C and 100°C and in the case Y=Br alternatively with pyridinium hydrobromide perbromide in acetic acid or with Br₂ and mercury triflate in trifluoroacetic acid. Iodination can be accomplished by treatment with ICl or I₂ and silvertriflate.

### Method B.

The compounds of formula I wherein R¹ and R² are H, X and Y being the same or different and selected from alkoxy, alkyl, alkylthio, fluoroalkyl, halogen, hydroxy, nitrile, OCF₃ and SCF₃ may be prepared from compounds of formula III, where PG are for example alkyl, 2-(trimethylsilyl)ethoxymethyl (SEM), methoxymethyl(MOM), 2-(methoxy)ethoxymethyl (MEM), benzyl (Bn) 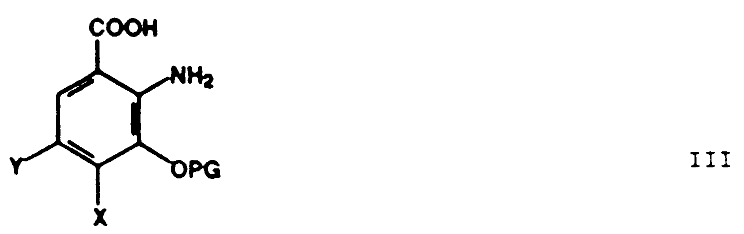
and X and Y are as defined in formula I in method B with the addition that hydroxy might be protected as OPG, for example by deprotecting with a Brφnsted acid such as 48% HBr, 55% HI or a Lewis acid such as BBr₃ or with alkylSNa or arylSNa followed by adjustment of the pH to obtain the free anthranilic acid derivative. In the case of PG=SEM, deprotection can be performed using tetrabutylammonium fluoride (TBAF) or CsF in suitable solvents such as N,N-dimethylpropyleneurea (DMPU) or N,N-dimethylformamide (DMF) at elevated temperature. A benzylgroup can be removed by hydrogenolysis using for example H₂ and Pd/C as catalyst.

### Method C.

The compounds of formula I wherein R¹ and R² are H, X and Y being the same or different and selected from alkoxy, alkyl, alkylthio, fluoroalkyl, halogen, hydroxy, nitrile, OCF₃ and SCF₃ may be prepared from compounds of formula IV 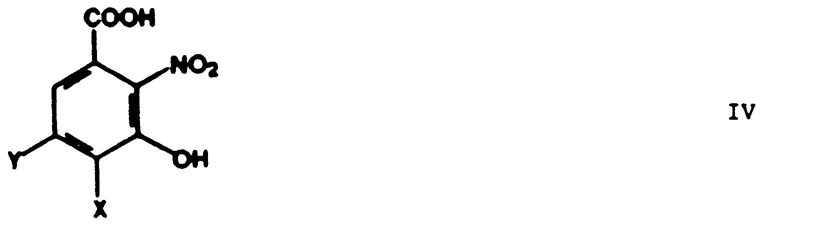
where X and Y are as defined in formula I in method C, for example by reducing with H₂ and a catalyst such as Pd or PtS₂ on carbon or Raney nickel between atmospheric and elevated pressure in a suitable solvent such as EtOH or EtOAc. The reduction can also be accomplished by reaction with SnCl₂ or NH₂NH₂ ·H₂O in a sutitable solvent such as EtOH.

### Method D.

The compounds of formula I, wherein R¹ and R² being the same or different and selected from H and alkyl, X = Cl, Br or I and Y is selected from alkoxy, alkyl, fluoroalkyl and halogen, may be prepared from compounds of formula V 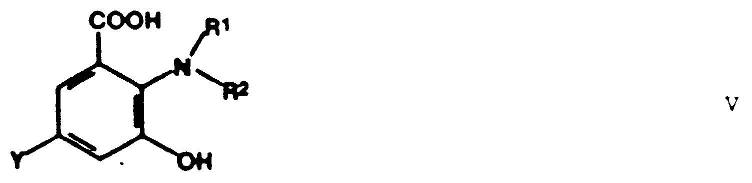
where R¹ , R², and Y are as defined in formula I in method D, for example by halogenating with Br₂ and HBr in acetic acid or Cl₂ and HCl in acetic acid at elevated temperature. Alternatively, V could be halogenated with Br₂ or I₂ and mercury triflate in trifluoroacetic acid at room or elevated temperature.

### Method E.

The compounds of formula I wherein R¹ = alkyl, R² = H or alkyl and X, Y being selected from alkoxy, alkyl, alkylthio, fluoroalkyl, halogen, hydroxy, nitrile, OCF₃ and SCF₃ may be prepared from compounds of formula VI 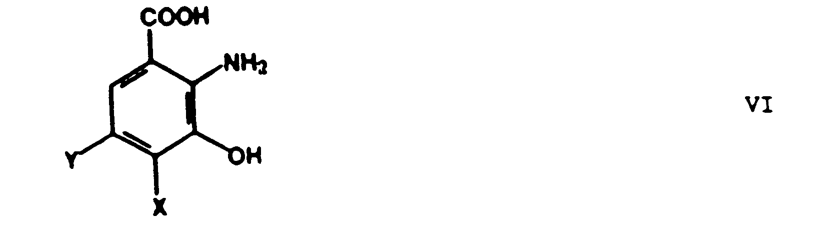
where X and Y are as defined in formula I in method E, for example by reductive alkylation with an aldehyde corresponding to R¹ and R² and reducing agents such as NaCNBH₃ and HCl in suitable solvents such as CH₃CN, H₂O or MeOH. Mono- and di-N alkylated derivatives can be separated for example by chromatography.

### Method F.

The compounds of formula I wherein R¹ and R² being the same or different and selected from H and alkyl, X and Y being the same or different and selected from alkoxy, alkyl, alkylthio, fluoroalkyl, halogen, hydroxy, nitrile, OCF₃ and SCF₃ may be prepared from compounds of formula VII 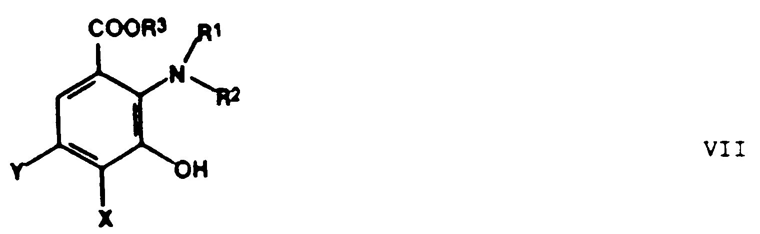
where R¹ , R², X and Y are as defined in formula I in method F and R³ selected from alkyl, benzyl, 1,1,1-trichloroethyl and SEM, for example by deesterifying with a base such as KOH in a suitable solvent such as MeOH at room temperature or elevated temperature, or by a Brønstead acid such as 48% HBr at elevated temperature, or by alkylSNa or arylSNa. In the case where R³ is benzyl, the carboxylic acid can be otbained by hydrogenolysis for example with H₂ and Pd/C. 1,1,1-Tricloroethylester can be cleaved for example with Zn in HOAc and SEM-ester for example with TBAF in DMPU.

### Intermediates

### Method II:a.

Compounds of the formula II wherein R¹ and R²=H can be prepared from compounds of formula VIII 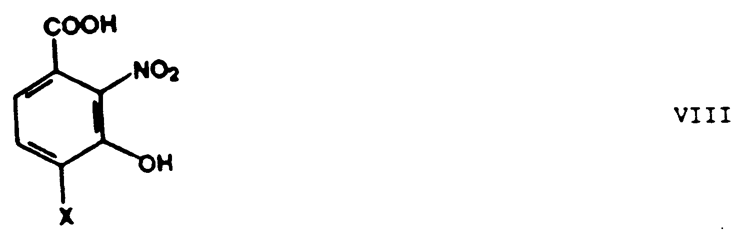
wherein X is selected from alkoxy, alkyl, alkylthio, fluoroalkyl, halogen, nitrile, OCF₃ and SCF₃ for example by reduction according to method C.

### Method II:b.

Compounds of the formula II can be prepared from compounds of formula IX 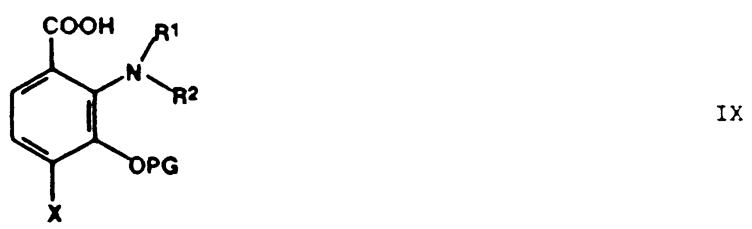
wherein R¹ and R² being the same or different and selected from H and alkyl, PG= alkyl, SEM, MEM, MOM, Bn and X is selected from alkoxy, alkyl, alkylthio, fluoroalkyl, halogen, hydroxy, nitrile, OCF₃ and SCF₃ according to method B.

### Method III:a.

The compounds of the formula III wherein PG=Me can be prepared from compounds of formula X 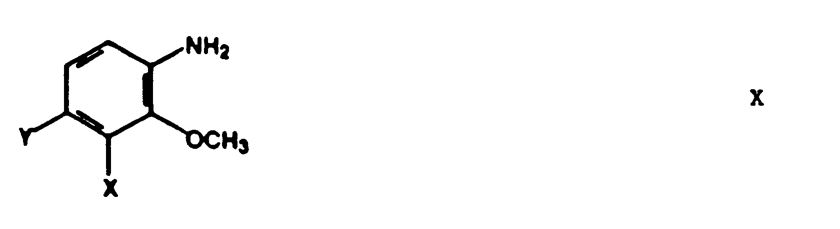
wherein X and Y being the same or different and selected from alkoxy, alkyl, fluoroalkyl, halogen, hydroxy (protected as OMe or OBn) and OCF₃ for example by reaction with chloralhydrate and NH₂OH·HCl and concentrated HCl at elevated temperature in water, optionally in the presence of Na₂SO₄, then with H₂SO₄ at elevated temperature and finally with NaOH and H₂O₂ at elevated temperature. The pH is adjusted at the end of this procedure to obtain the free anthranilic acid derivative.

### Method III:b.

The compounds of formula III can be prepared from compounds of formula XI, DMG = COtBu, CO₂tBu, PG = alkyl, SEM, MEM, MOM 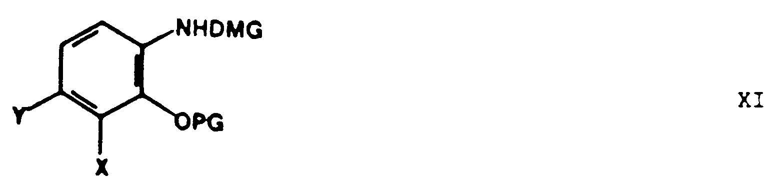
wherein X and Y being selected from alkoxy, alkyl, alkylthio, chloro, fluoro, fluoroalkyl, hydroxy (protected as OPG ) and OCF₃ for example by reaction with alkyllithium in a suitable solvent such as tetrahydrofuran (THF) at low temperature. The alkyllithium derivative is then reacted with CO₂ (s), acidified and the DMG group is removed by aqueous HCl at elevated temperature.

### Method III:c.

The compounds of formula III can be prepared from compounds of the formula XII
PG = Me, SEM, MOM, MEM 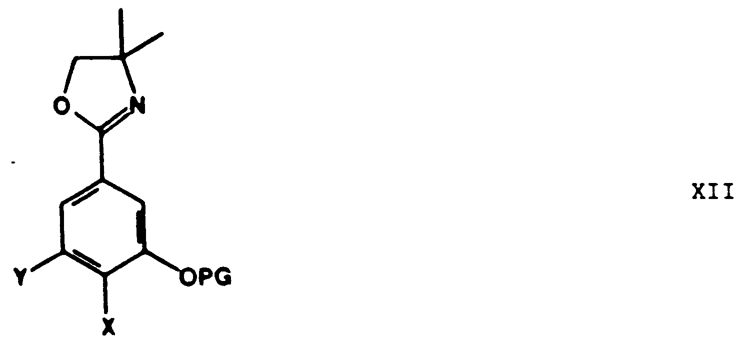
wherein X and Y being selected from alkoxy, alkyl, alkylthio, chloro, fluoro, fluoroalkyl, hydroxy (protected as OPG ) and OCF₃ for example by reaction with alkyllithium at low temperature in a suitable solvent such as THF. The aryllitium derivative is then reacted with either trimethylsilymetyl azide (TMSCH₂N₃) or with an azide such as tosylazide followed by reduction to the corresponding amine. The oxazolidine can be hydrolysed with aqueous HCl (followed by adjustment of the pH) to the anthranilic acid derivatives.

### Method III:d.

The compounds of formula III can be prepared from compounds of formula XIII
PG = alkyl, SEM, MEM, MOM 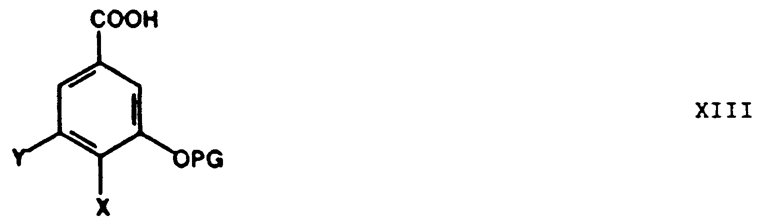
wherein X and Y being the same or different and selected from alkoxy, alkyl, alkylthio, fluoroalkyl, halogen, hydroxy, nitrile, OCF₃ and SCF₃ for example by reaction with for example HNO₃ at temperatures between room temperature and 100°C followed by reduction of the nitro group according to method C.

### Method IV: a.

Compounds of formula IV can be prepared from compounds of formula XIV 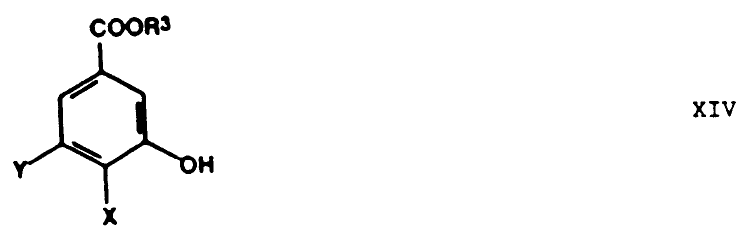
wherein X, Y being the same or different and selected from alkoxy, alkyl, alkylthio, fluoroalkyl, halogen, hydroxy, nitrile, OCF₃ and SCF₃ and R³ = H or alkyl for example by nitration according to method III:d. In the case where R³ = alkyl, hydrolysis to the carboxylic acid can be performed by a base such as KOH or by concentrated HCl or HBr at elevated temperature.

### Method IV:b.

Compounds of formula IV can be prepared from compounds of formula XV 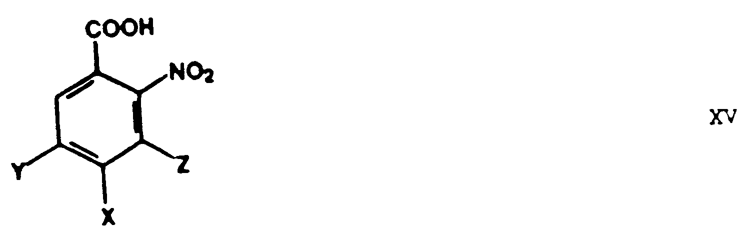
wherein X is selected from alkoxy, alkyl, alkylthio, halogen, hydroxy ( protected as OPG ), OCF₃, SCF₃ and Y is selected from alkoxy, alkyl, alkylthio, fluoroalkyl, hydroxy ( protected as OPG ), OCF₃, SCF₃ and Z = halogen, NHCOR or OCOR ( R=alkyl ) for example by reaction with a base such as KOH in water or polar aprotic solvents such as dimethylsulfoxide (DMSO) or DMF between room temperature and reflux. In the presence of a protecting group, said group can be removed according to method B.

### Method V: a.

The compounds of formula V wherein R¹ and R² are H or alkyl, and Y = Br, Cl, I can be prepared from compounds of formula XVI 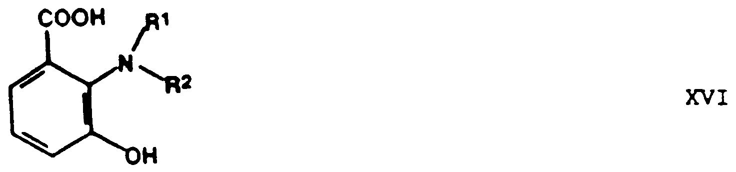
for example by halogenation according to method A.

### Method V: b.

Compounds of formula V can be prepared from compounds of formula XVII 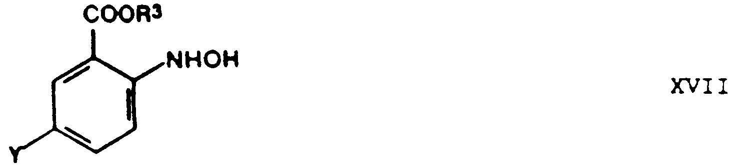
wherein R³ is alkyl and Y is selected from alkoxy, alkyl and fluoroalkyl for example by treatment first with TsCl and a base such as Et₃N in a solvent such as CHCl₃ or THF at elevated temperature giving the phenoltosylate, then detosylated for example with NaC₁₀H₈ in THF at low temperature, followed by purification by chromatography and finally hydrolysis of the ester, followed by adjustment of the pH to obtain the free anthranilic acid derivative.

### Method VII:a.

Compounds of formula VII wherein R¹, R², X and Y are as defined in formula I in method A and R³ are as defined in method F can be prepared from compounds of formula XVIII 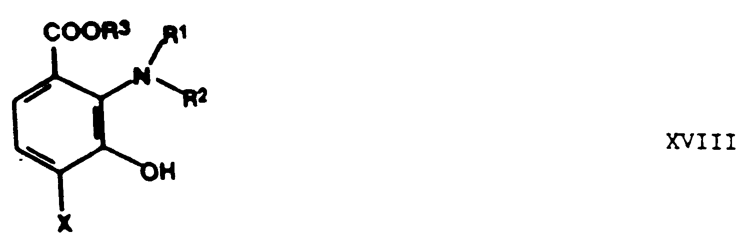
for example by halogenation according to method A.

### Method VII:b.

Compounds of formula VII wherein R¹ and R² are H and X and Y are as defined in formula I in method B and R³ is the same as defined in method F can be prepared from compounds of formula XIX 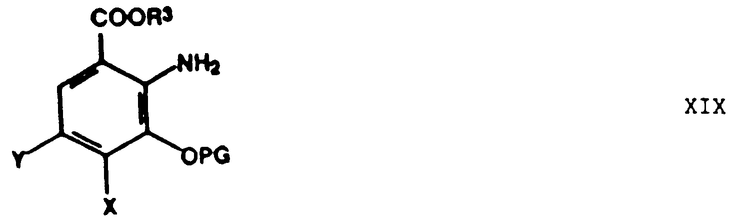
for example by deprotection according to method B.

### Method VII:c.

Compounds of formula VII wherein R¹ and R² are H and X and Y are as defined in formula I in method C and R³ is the same as defined in method F can be prepared from compounds of formula XX 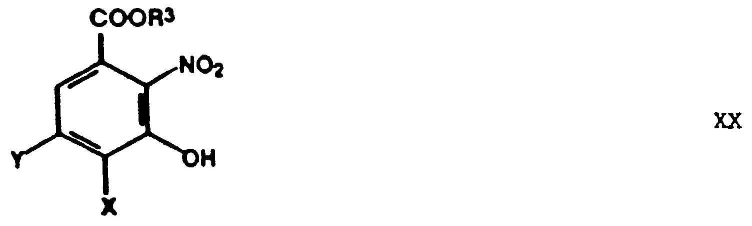
for example by reduction according to method C.

### Method VII:d.

Compounds of formula VII wherein R¹, R², X and Y are as defined in formula I in method D and R³ is the same as defined in method F can be prepared from compounds of formula XXI 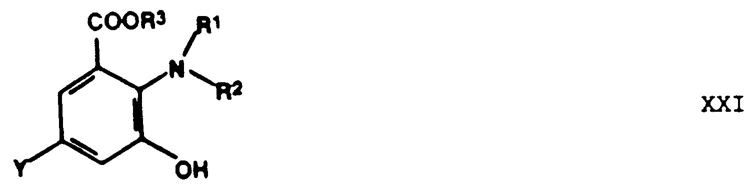
for example by halogenation according to method D.

### Method VII:e.

Compounds of formula VII wherein R¹, R², X and Y are as defined in formula I in method E and R³ is the same as defined in method F can be prepared from compounds of formula XXII 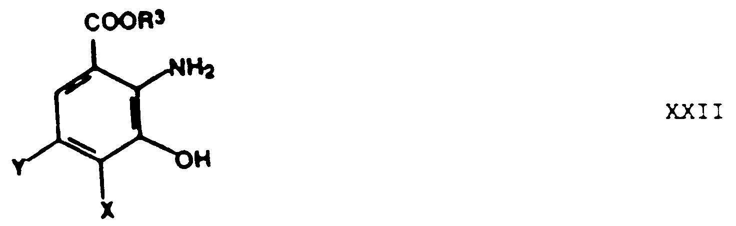
for example by alkylation according to method E.

### Working examples

### Example 1 (Method A)

### 5-Bromo-4-chloro-3-hydroxyanthranilic acid

4-Chloro-3-hydroxyanthranilic acid [1] (50mg, 0.27mmol) was dissolved in HOAc (2.0ml). A solution of 47% aqueous HBr (0.02ml, 0.54mmol) was added, precipitating the amino acid. Addition of Br₂ (86mg, 0.54mmol) in HOAc (3ml) made the hydrobromide almost completely dissolved and then precipitated again. The reaction mixture was stirred for 2 days at room temperature under nitrogen atmosphere. The acetic acid was evaporated with toluene and the crude product was recrystallized from 40% EtOH. Treating the precipitate dissolved in MeOH with Charcoal, followed by evaporation of the solvent, gave 5-bromo-4-chloro-3-hydroxyanthranilic acid (37mg), m.p. 227-8°C (dec.). ¹H NMR(CD₃OD):δ 7.72(s,1H), 5.0(br,4H). ¹³C NMR(CD₃OD):δ 170.27, 143.51, 143.10, 127.16, 125.41, 111.04, 106.73. MS(TSP) :m/z(rel.int.)270/268/266(M⁺,0.28/1.0/0.80).

### Example 2 (Method A)

### 2-Methoxy-3-trifluoromethylaniline hydrochloride

2-Trifluoromethylanisole [2] (1.00g, 5.7mmol) was dissolved in dry THF (90ml). The solution was cooled to -75°C and t-BuLi (5.0ml, 1.3M in pentane, 6.5mmol) was added dropwise during 5 minutes. The reaction was kept at -75°C for 1 hour, then the temperature was raised to -20°C for 2 hours and finally to 0°C for 1 hour. The solution was cooled to -5°C and TMSCH₂N₃ (0.94ml, 6.5mmol) was added dropwise. After 4 hours at room temperature, H₂O (1.2ml) was added and the solvent was evaporated. The residue was partitionated between Et₂O (50ml) and H₂O (10ml), the aqueous phase was extracted with Et₂O (10ml) and the combined ether phases were washed with brine and dried (Na₂SO₄). The aniline was precipitated as salt through the addition of HCl (3M in Et₂O) giving 2-methoxy-3-trifluoromethylaniline hydrochloride (598mg). ¹H NMR(CD₃OD):δ 7.80(d,J=7.9Hz,1H), 7.75(d,J=8.1Hz,1H), 7.51(t,J=8.0Hz,1H), 5.0(br,1H), 4.03(s,3H). ¹³C NMR(CD₃OD):δ 153.10, 130.30, 129.50, 128,20(q,J=5Hz), 127.27, 127.06(q,J=31Hz), 125.04(q,J=273Hz), 64.37. MS(EI,70eV):m/z(rel.int.) 191(M⁺,0.76)

### 2-Methoxy-3-trifluoromethylpivaloyl anilide

2-Methoxy-3-trifluoromethylaniline hydrochloride (264mg, 1.4mmol) was dissolved in CH₂Cl₂ (9.0ml), IM NaOH (5.0ml) and pivaloylchloride (255µl, 2.7mmol) was added and the reaction mixture was stirred for 2 days at room temperature. The mixture was diluted with CH₂Cl₂ (60ml) and H₂O (15ml), the organic layer was washed with IM NaOH (20ml) and brine (20ml), followed by drying (Na₂SO₄). The crude product was purified by chromatography (SiO₂, CHCl₃-MeoH 45:1) giving 2-methoxy-3-trifluoromethylpivaloyl anilide (295mg). ¹H NMR(CDCl₃): δ 8.51(d,J=8.1Hz,1H), 7.96(br,1H), 7.8(d,J=8.1,1H), 7.18(t,J=8.0Hz,1H), 3.83(s,3H), 1.31(s,9H). ¹³C NMR-(CDCl₃) :δ 177.42, 147.68, 133.69, 125.44, 125.33, 124.23(q,J=31Hz), 123.94(q,J=273Hz), 121.84(q,J=5Hz), 62.51, 40.27, 27.75. MS(EI,70eV):m/z(rel.int.) 275-(M⁺,0.12)

### 3-Methoxy-4-trifluoromethylpivaloylamidoanthranilic acid

2-Methoxy-3-trifluoromethylpivaloyl anilide (385mg, 1.4mmol) was dissolved in dry THF (50ml) and cooled to -75°C. t-BuLi (2.7ml, 1.3M in pentane, 3.5mmol) was added dropwise at -75°C to -73°C. After the addition, the temperature was raised to -50°C for 0.5 hour followed by 2.5 hours at -20°C. The solution was cooled and transferred into a slurry of dry-ice in Et₂O (≈10g in 80 ml). The reaction mixture was allowed to warm up to room temperature, H₂O (2ml) was added and the solvent was evaporated. The residue was partitionated between Et₂O (90ml) and H₂O (10ml). The ether phase was extracted with H₂O and the combined aqueous phases were washed with Et₂O and acidified to pH 3 with 2M HCl precipitating the product. The residue was concentrated and more of the product precipitated, giving after vacuum drying a total of 3-methoxy-4-trifluoromethylpivaloylamidoanthranilic acid (182mg), m.p. 166.5-7.5°C. ¹H NMR(CD₃OD):δ 7.86 (d,J=8.2Hz, 1H), 7.68(d,J=8.4Hz,1H), 4.92(br,2H), 3.86(s,3H), 1.41(s,9H). ¹³C NMR(CD₃OD):δ 180.88, 169.01, 156.73, 135.78, 134.40, 129.13(q,J=31Hz), 127,34, 125.89(q,J=5Hz), 125.05(q;J=273Hz, 62.78, 40,49, 27,90. MS(TSP):m/z(rel.int.) 320(M⁺ ,0.27).

### 3-Methoxy-4-trifluoromethylanthranilic acid

3-Methoxy-4-trifluoromethylpivaloyamidoanthranilic acid (190mg) was mixed with 6M HCl (80ml) and heated at 100°C for 8 hours and left to cool to room temperature over night. Azeotropic evaporation with toluene and vacuum drying gave a mixture of free base and the HCl-salt of 3-methoxy-4-trifluoromethylanthranilic acid (159mg). ¹ H NMR(CD₃OD):δ 7.83(d,J=9.0Hz,1H), 6.92(d,J=8.5,1H), 5.0(br,3H), 3.89(s,3H). ¹³C NMR(CD₃OD):δ 160.03, 140.30, 131.07, 119.38(q,J=31Hz), 119.19, 115.25(q,J=273Hz), 112.37, 109.07(q,J=5Hz), 53.14. MS(EI,70eV):m/z(rel.int.) 235(M⁺,0.98)

### 3-Hydroxy-4-trifluoromethylanthranilic acid

3-Methoxy-4-trifluoromethylantranilic acid (43mg, 0.18mmol) was mixed with freshly distilled 48% HBr (5ml) and heated at 100°C for 14 hours. Water and HBr were evaporated and the crude product was dissolved in boiling water (3ml), treated with charcoal, filtered followed by evaporation. The residue was dissolved in EtOH and diisopropyl ether was added to precipitate 3-hydroxy-4-trifluoromethylanthraninlic acid (18mg). M.p. 200-2°C (dec.). ¹H NMR(DMSO-d₆):δ 7.42(d,J=8.8Hz,1H), 6.69 (d,J=8.8Hz,1H), 3.33(br,4H). MS(EI,70eV):m/z(rel.int.) 221(M⁺,0.77)

### 5-Bromo-3-hydroxy-4-trifluoromethylanthranilic acid

3-Methoxy-4-trifluoromethylanthranilic acid (79mg, 0.30mmol) was demethylated with freshly distilled 48% HBr (10ml) at 100°C for 15 hours. After evaporation followed by vacuum drying the salt was mixed with HOAc (4ml) and Br₂ (96mg, 0.60mmol) in HOAc (3ml) was added dropwise. The reaction mixture, protected from light, was left at room temperature under argon for 2 days and at 40°C for 33 hours. After evaporation the crude product was recrystallized from EtoH-H₂O (1.5:1) giving 5-bromo-3-hydroxy-4-trifluoromethylanthranilic acid (58mg). M.p. 199-200°C (dec). ¹ H NMR(CD₃OD):δ 7.80(s,1H), 5.0(br,4H). ¹³C NMR(CD₃OD):δ 170.35, 146.26, 146.07, 130.24, 125.44(q,J=276Hz), 121.04(q,J=29Hz), 113.73, 102.52. MS(EI, 70eV):m/z(rel.int) 301/299(M⁺, 0.84/0.83).

### Example 3 (Method A)

### 4,5-Dichloro-3-hydroxyanthranilic acid

4-Chloro-3-hydroxyanthranilic acid [1] (150mg, 0.80mmol) was dissolved in HOAc (12ml) and flushed with argon. Hydrochloric acid (336µl,12M, 4.8mmol) was added, and then a solution of Cl₂ in HOAc (1.78ml, 70mg/ml, 1.76mmol) was added dropwise. The reaction was left at room temperature under argon, protected from light, for 20 hours. The precipitated product was filtered off and dried in vacuum. The crude product was recrystallized from EtOH-H₂O 1:1 yielding 4,5-dichloro-3-hydroxyanthranilic acid (14mg). ¹H NMR(CD₃OD):δ 7.57(s,1H), 4.98(br,4H). ¹³C NMR(CD₃OD):δ 170.70, 143.58, 143.36, 124.00, 118.68, 110.63, 109.46. MS(TSP):m/z(rel.int.) 224/222(M⁺0.53/1.0).

### Example 4 (Method A)

### 5-Bromo-3-hydroxy-4-methylanthranilic acid

To 3-hydroxy-4-methylanthranilic acid [3] (251mg, 1.5mmol) in HOAc (20ml) was added Br₂ (719mg, 4.5mmol) and the reaction mixture was stirred over night at room temperature. The product was filtered off and washed with cooled HOAc to give 5-bromo-3-hydroxy-4-methylanthranilic acid (349mg). ¹H NMR(DMSo-d₆):δ 9.50(br,4H), 7.54(s,1H), 2.26(s,3H).
¹³CNMR(DMSO-d₆):δ 168.06, 145,61, 134.84, 130.02, 125.45, 114.55, 17.59. MS(EI, 70eV):m/z(rel.int.)247/245(M⁺, 0.77/0.78).

### Example 5 (Method A)

### 2-(Ethylthio)-6-nitrophenol

NaNO₃( 3.13g, 36.8mmol) and La(N0₃)3*6H₂O( 0.145g, 0.33mmol) were dissolved in water( 26ml) and concentrated HCl( 26ml). To this solution were added 2-(ethylthio)phenol( 5.16g, 33.5mmol) dissolved in diethylether( 105ml). The reaction mixture was then stirred at room temperature for 20 hours and then extracted with chloroform and washed with water. After drying( MgSO₄) and evaporation the crude product ( 6.45g) was purified by flash chromatography on SiO₂. Eluation with hexane-ethyl acetate afforted the product( 2.23g). An analytical pure sample was obtained by crystallisation from ethyl acetate-hexane, mp= 55.5-57°C. ¹H NMR(CDCl₃): δ 8.54(dd, J₁=8.5Hz, J₂=1.7Hz, 1H), 7.55(dd, J₁=7.6Hz, J₂=1.5Hz, 1H), 6.96(dd, J₁=8.6Hz, J₂=7.6Hz, 1H), 2.98(q, J=7.3Hz, 2H), 1.34(t, J=7.3Hz, 3H). ¹³C NMR: δ 153.53, 136.80, 134.13, 129.19, 122.75, 120.42, 26.86, 14.51. MS(EI, 70eV): m/z(rel. int.) 199(M+, 0.9)

### 2-(Ethylthio)-6-nitroanisole

2-(Ethylthio)-6-nitrophenol( 0.7g, 3.5mmol) was dissolved in DMF( 9ml). MeI( 0.43ml, 6.9mmol) and K₂CO₃(0.94g, 6.8mmol) were added and the reaction mixture was stirred at room temperature for 18 hours. After evaporation, ethyl acetate was added to the residue and the salts were filtered of. Evaporation again gave a crude product (0.98g) which was purified by flash chromatography on SiO₂. Eluation with hexane-ethyl acetate afforted the product( 0.45g). ¹H NMR(CDCl₃): δ 7.53(dd, J=8.0Hz, 1H), 7.40(dd, J=8.0Hz, 1H), 7.14(dd, J=8.0Hz, 1H), 3.94(s, 3H), 2.93(q, J=7.4, 2H), 1.32(t, J=7.4Hz, 3H). ¹³C NMR(CDCl₃): δ 150.87, 144.98, 136.04, 132.27, 124.90, 122.03, 62. 35, 26.55, 14.32. MS(EI, 70eV): m/z(rel int.) 213(M+, 1.0)

### 3-(Ethylthio)-2-methoxyaniline

A mixture of 2-(ethylthio)-6-nitroanisole( 0.59g, 2.77mmol) and SnCl₂,2H₂O(6.2g, 27mmol) in ethanol( 50ml) was heated at reflux temperature for 2 hours under nitrogen atmosphere. After cooling, water was added and the pH was made slightly basic (pH 7-8) by adding sodium bicarbonate ( 5% aq) before extraction with ethyl acetate. Drying(MgSO₄) and evaporation gave a crude product( 0.48g) which was purified by flash chromatography on SiO₂. Eluation with hexan-ethyl acetate afforted the product( 0.38g). ¹H NMR(CDCl₃): δ 6.88(m, 1H), 6.63(dd, J=7.9Hz, 1H), 6.56(dd, J=7.9Hz, 1H), 3.82(s, 3H), 2.92(q, J=7.3Hz, 2H), 1.33(t, J=7.3Hz, 3H). ¹³C NMR(CDCl₃). δ 145.11, 140.74, 131.24, 125.52, 118.13, 114.07, 59.44, 26.56, 14.79. MS(EI, 70eV): m/z(rel.int.) 183(M+, 1.0)

### N-(3-ethylthio-2-methoxyphenyl)-pivaloylic amide

3-(Ethylthio)-2-methoxyaniline( 0.25g, 1.36mmol) was dissolved in dichloromethane( 10ml). Pivaloylchloride( 0.28ml, 2.35mmol) and triethylamine( 0.33ml, 2.36mmol) where added and the mixture was heated at reflux temperature under nitrogen atmosphere for 20 hours. Evaporation gave a crude product( 0.61g) which was purified by flash chromatography on SiO₂. Eluation with hexane-ethyl acetate gave the product( 0.3g). ¹H NMR(CDCl₃): δ 8.18(dd, J₁=8.1Hz, J₂=1.5Hz, 1H), 8.08(br, 1H), 7.03(dd, J=8.0Hz, 1H), 6.94(dd, J₁=8.0Hz, J₂=1.5Hz, 1H), 3.83(s, 3H), 2.90(q, J=7.5Hz, 2H), 1.30(s, 9H), 1.29(t, J=7.5Hz, 3H). ¹³NMR (CDCl₃): δ 177.04, 147.07, 132.73, 130.26, 125.61, 123.92, 118.31, 60.55, 40.52, 28,13, 26.85, 14.76. MS(EI, 70eV): m/z(rel. int.) 267(M+, 1.0)

### N-(3-ethylthio-6-methoxycarbonyl-2-methoxyphenyl)-N-methoxycarbonyl-pivaloylic amide

t-Butyllithium( 3ml, 1.5M in pentane, 4.4gmmol) was added to a solution of N-(3-ethylthio-2-methoxyphenyl)-pivaloylic amide( 0.352g, 1.32mmol) in dry THF( 6ml) under nitrogen at -78°C, and the stirred reaction mixture was allowed to slowly reach -20°C. After 4.5 hours methyl chloroformate( 0.51ml, 6.6mmol) was added and then the reaction was allowed to reach room temperature. After 20 hours water was added and the reaction mixture extracted with ethyl acetate. Evaporation of the organic phase gave a crude product( 0.51g) which was purified by flash chromatography on SiO₂. Eluation with hexane-ethyl acetate afforted the product(40mg). ¹H NMR(CDCl₃): δ 7.77(d, J=8.4Hz, 1H), 7.17(d, J=8.4, 1H), 3.82(s, 3H), 3.75(s, 3H), 3,70(s, 3H), 2.97(q, J=7.4Hz, 2H), 1.42(s, 9H), 1.32-1.41(m, 3H). ¹³C NMR(CDCl₃): δ 183.66, 165.64, 155.23, 153.09, 139.98, 135.09, 127.78, 125.01, 124.92, 60.88, 54.3g, 52.72, 43.98, 28.32, 25.63, 14.16. MS(EI, 70eV): m/z(rel.int.) 383(M+, 0.42)

### 4-(Ethylthio)-3-hydroxyanthranilic acid

N-(3-ethylthio-6-methoxycarbonyl-2-methoxyphenyl)-N-methoxycarbonyl-pivaloylic acid amide ( 0.077g, 0.20mmol) was dissolved in 48% aqueous HBr and heated at reflux temperature for 3 hours. Evaporation gave a product (0.06g) which was purified by flash chromatography on SiO₂. Eluation with toluene-ethyl acetate afforted the product( 9mg). ¹H NMR(CD₃OD): δ 7.17(d, J=8.4HZ, 1H), 6.40(d, J=8.4Hz, 1H), 2.63(q, J=7.4Hz, 2H), 1.03(t, J=7.3Hz, 3H). MS(TSP): m/z(rel.int.) 214(M+1, 1.0)

### 5-Bromo-4-(ethylthio)-3-hydroxyanthranilic acid

4-(ethylthio)-3-hydroxyanthranilic acid( 0.007g, 0.03mmol) was dissolved in acetic acid( 0.3ml). Bromine( 0.0025ml, 1.5mmol) was added and the stirred reaction mixture was left at room temperature 5 hours. Evaporation of the solvent and excess of bromine gave the product( 10mg). MS( EI, 70eV): m/z(rel.int.) 291/293(M+, 0.65/0.68)

### Example 6 (Method C)

### Ethyl 4-bromo-3,5-diethoxybenzoate

4-Bromo-3,5-dihydroxybenzoic acid [4] (4.68g, 20mmol), potassium carbonate (8.28g, 60mmol) and diethyl sulphate (7.84ml, 60mmol) in dry acetone (50ml) under nitrogen atmosphere was stirred at room temperature for 20 minutes and then refluxed for 8 hours. The salts were separated by filtration and the filtrate was evaporated under reduced pressure. The residue was dissolved in ether and successively washed with water, aqueous 5% sodium hydroxide, water, concentrated ammonium hydroxide, water, dilute hydrochloric acid and water and finally dried (Na₂SO₄). Removal of the ether gave ethyl 4-bromo-3,5-diethoxybenzoate (2.63g). ¹H NMR(CDCl₃):δ 7.22(s,2H), 4.39(q,J=7.1Hz,2H), 4.18(q,J=7.0Hz,4H), 1.50(t,J=7.0Hz,6H), 1.41(t,J=7.1Hz, 3H). ¹³C NMR(CDCl₃):δ 166.75, 157.02, 130.78, 107.91, 106.81, 65.42, 61.60, 14.74, 14.41

### 4-Bromo-5-ethoxy-3-hydroxybenzoic acid

Ethyl 4-bromo-3,5-diethoxybenzoate (1.015g, 3.2mmol) in dimethylformamide (5ml) was added to sodium ethanethiolate (4.53g, 51.2mmol) in dimethylformamide (25ml) under nitrogen atmosphere. The mixture was stirred at 100°C for 8 hours. After cooling, it was added to water, acidified with 1M HCl and extracted with ether, re-extracted with aqueous sodium bicarbonate, acidified with 1M HCl and extracted with ether. The organic layer was dried (Na₂SO₄) and concentrated to dryness. Purification by flash columnchromatography (SiO₂,Toluene-HOAc 5:1) gave 4-bromo-5-ethoxy-3-hydroxybenzoic acid (115mg). ¹H NMR(DMSO-d₆):δ 7.13(d,J=1.8Hz,1H), 6.95(d,J=1.8Hz,1H), 4.08(q,J=7.0Hz,2H), 3.4(br,2H), 1.30(t,J=7.0Hz,3H). ¹³C NMR(DMSO-d₆):δ 167.45, 156.48, 155.81, 131.32, 109.67, 104.47, 104.29, 64.65, 14.57. MS(TSP):m/z 263/261(M⁺1).

### Methyl 4-bromo-5-ethoxy-3-hydroxybenzoate

4-Bromo-5-ethoxy-3-hydroxybenzoic acid (115mg, 0.44mmol) in dry methanol (10ml) was saturated with hydrogen chloride gas at 0°C and the reaction was then stirred at room temperature over night. After concentration to dryness the residue was dissolved in chloroform and washed with aqueous sodium bicarbonate, water and brine. The organic layer was dried over sodium sulfate and concentrated to dryness giving methyl 4-bromo-5-ethoxy-3-hydroxybenzoate (94mg). ¹H NMR(CDCl₃):δ 7.35 (d,J=1.8Hz,1H) 7.14(d,J=1.8Hz,1H), 5.85(br,1H), 4.17(q,J=7.0Hz,2H), 3.91(s,3H), 1.49(t,J=7.0Hz,3H). ¹³C NMR(CDCl₃):δ 167.15, 156.52, 154.38, 130.84, 110.07, 106.03, 105.47, 65.39, 52.70, 14.70. (MS(TSP):m/z 277/275(M⁺1).

### Methyl 4-bromo-5-ethoxy-3-hydroxy-2-nitrobenzoate

A solution of methyl 4-bromo-5-ethoxy-3-hydroxybenzoate (89.7mg, 0.326mmol) in a nitromethane-dichloromethane mixture 1:1 (2.0ml) was stirred with 90% nitric acid (15µl) at 40°C for 10 minutes. Ice was added, the organic layer separated and the aqueous layer extracted with chloroform. The combined organic layers were washed with water and concentrated. Purification by flash columnchromatography (SiO₂,toluene-ethyl acetate 10:1) gave methyl 4-bromo-5-ethoxy-3-hydroxy-2-nitrobenzoate (37mg). ¹H NMR(CDCl₃): 6.59(s,1H), 4.21(q,J=7.0Hz,2H), 3.90(s,3H), 1.47(t,J=6.9Hz,3H). ¹³C NMR(CDCl₃): 167.12, 162.20, 154.36, 132.25, 126.89, 105.35, 103.26, 66.63, 53.94, 14.61. MS(TSP):m/z 339/337(M+NH₄⁺).

### 4-Bromo-5-ethoxy-3-hydroxy-2-nitrobenzoic acid

Methyl 4-bromo-5-ethoxy-3-hydroxy-2-nitrobenzoate (30.2mg, 0.094mmol) was dissolved in ethanol (1.2ml) and potassium hydroxide (62mg) in water (0.54ml) was added. The reaction mixture was stirred at 40° for 16 hours. After cooling, the solution was acidified with hydrochloric acid to pH 2 and then concentrated to dryness. The residue was triturated with water, filtered, washed with water and dried giving 4-bromo-5-ethoxy-3-hydroxy-2-nitrobenzoic acid (24.6mg). ¹H NMR(CD₃OD):δ 6.82(s,1H), 4.74(br,2H), 4.00(q,2H J=7.0Hz,2H), 1.26(t,3H J=7.0Hz,3H). ¹³C NMR(CD₃OD):δ 167.31, 159.73, 150.96, 135.28, 128.19, 106.87, 106.30, 67.42, 15.38. MS(EI, 70eV): m/z(rel.int.)307/305(M⁺, 0.99/1.00)

### 4-Bromo-5-ethoxy-3-hydroxyanthranilic acid

A mixture of 4-bromo-5-ethoxy-3-hydroxy-2-nitrobenzoic acid (19.8mg, 0.065mmol) and SnCl₂,2H₂O (73mg) in ethanol (2.0ml) was heated at 70° for 5.5 hours under nitrogen atmosphere. After cooling, water (2.0ml) was added and the pH was made slightly basic (pH 7-8) by adding sodium bicarbonate (5% aq) before extraction with ethyl acetate. The organic phase was washed with brine and dried over sodium sulphate. Evaporation of the solvent gave 4-bromo-5-ethoxy-3-hydroxyanthranilic acid (8.4mg). ¹ H NMR(CD₃OD):δ 6.93(s,1H), 4.80(br,4H), 3.89 (q,J=7.0Hz,2H), 1.30(t,J=7.0Hz,3H). ¹³C NMR(CD₃OD):δ 171.73, 147.72, 145.07, 137.91, 109.46, 108.72, 107.25, 67.05, 15.71. MS(TSP):m/z 278/276(M+1).

### Example 7 (Method D)

### 5-Bromo-3-hydroxyanthranilic acid

To 3-hydroxyanthranilic acid [4] (5g, 32.6 mmol) mixed with acetic acid (450ml), Br2 (10.4g, 65.2mmol) in acetic acid (50ml) was added dropwise. The slurry was stirred for 1 hour at room temperature and then evaporated. The residue was dissolved in methanol (60ml) and precipitated with water (300ml). Filtration and drying gave 5-bromo-3-hydroxyanthranilic acid (7.38g), m.p. 210°C. ¹H NMR (DMSO-d₆) :δ 10.20(br,1H), 8.5(br,1H), 7.26(d,J=2.4Hz,1H), 6.84(d,J=2.2Hz,1H), 3.4(br,2H). ¹³C NMR(DMSO-d₆):δ 168.92, 146.27, 140.99, 123.14, 118.92, 111.02, 104.31. MS (EI,70ev):m/z(rel.int.) 233/231(M⁺,0.14/0.13).

### 4,5-Dibromo-3-hydroxyanthranilic acid

5-Bromo-3-hydroxyanthranilic acid(7.38g, 31.8mmol) was dissolved in methanol (120ml) and dichloromethane (250ml) saturated with hydrogenbromide was added, followed by addition of dichloromethane (300ml). After 48 hours at room temperature the solvent was evaporated. The residue (8.92g) was dissolved in acetic acid (400ml). Hydrogen bromide (100ml,48%,aq) and bromine (5.50g, 34.4mmol) in acetic acid (50ml) was added dropwise during 20 minutes at room temperature. The temperature was raised to 70°C and additional bromine (5.50g, 34.4mmol) in acetic acid (50ml) was added. After 14 hours at 90°C the solvent and excess of bromine were evaporated to give 20g of crude product which was dissolved in methanol (45ml) and precipitated with water (300ml). Filtration and drying gave 4,5-dibromo-3-hydroxyanthranilic acid (8.45g). M.p. 223°C(dec). ¹H NMR(DMSO-d₆):δ 9.3(Br,1H), 5.3(Br,1H), 7.56(s,1H). ¹³C NMR(DMSO-d₆):δ 168.67, 143.18, 142.64, 125.91, 117.83, 110.24, 106.98.MS(EI,70eV):m/z(rel.int.) 313/311/309(M⁺,0.51/1.0/0.51)

### 4,5-Dibromo-3-hydroxyanthranilic acid hydrochloride

4,5-Dibromo-3-hydroxyanthranilic acid (7.00g, 22.5mmol) was heated in MeOH (85ml), filtered and HCl/Et2O (≈10ml) and Et2O (120ml) was added. The hydrochloride was filtered off and the filtrate was concentrated. Ether was added to precipitate more product. The total amount was washed with ether and dried giving 4,5-dibromo-3-hydroxyanthranilic acid hydrochloride (5.38g). ¹H NMR(DMSo-d6):δ 7.56(s,1H), 6.0(br,5H). ¹³C NMR(DMSO-d₆):δ 168.89, 143.53, 142.54, 125.86, 117.90, 110.31, 107.20. MS(EI,70eV):m/z 313/311-/309(M⁺,0.51/1.0/0.50).

### Example 8 (Method F)

### Methyl 4-bromo-3-hydroxy-5-methylanthranilate

Methyl 3-hydroxy-5-methylanthranilate hydrochloride [5] (50mg, 0.23mmol) was dissolved in acetic acid (2.5ml), flushed with argon and bromine (36mg, 0.23mmol) in acetic acid (2.5 ml) was added dropwise. After 3 hours at room temperature, protected from light, additional bromine (18mg, 0.11mmol) in acetic acid (1ml) was added and left at that temperature over night. Acetic acid and excess bromine were evaporated under reduced pressure and coevaporated with water (2x5ml) and methanol(3x5ml) to dryness. Purification by preparative thinlayer chromatography(SiO₂) using first chloroform-methanol-ammonia (300:10:1) and secondly chloroform-methanol (1000:1) as eluent gave methyl 4-bromo-3-hydroxy-5-methylanthranilate (9mg). ¹H NMR(DMSO-d₆):δ 7.22(s,1H), 6.25(br,3H), 3.74(s,3H), 2.17(s,3H). MS(EI,70eV):m/z-(rel (.int.)261/259(M⁺,0.85/0.86).

### 4-Bromo-3-hydroxy-5-methylanthranilic acid

Methyl 4-bromo-3-hydroxy-5-methylanthranilate (14mg, 0.05mmol) was dissolved in EtOH (0.7ml) and flushed with argon. Aqueous KOH-solution (0.35ml, 0.05mmol) was added and the reaction was kept for 6 hours at 40°C protected from light. After acidification with 2M HCl to pH 2, water was evaporated and the residue was triturated with ice-water (0.1ml) and concentrated to dryness. Purification on preparative HPLC(Lichrosorb-C₁₈, MeOH-0.05M NH₄OAc(aq), 40:60), yielded 4-bromo-3-hydroxy-5-methylanthranilic acid (2mg). ¹H NMR(DMSO-d₆):δ 7.23(s,1H), 3.35(br,2H), 2.13(s,2H), 2.05(s,3H). MS(EI,70eV):m/z(rel.int.) 247/245(M⁺, 0.82/0.86).

### Example 9 (Method D)

### 5-Bromo-3-hydroxy-N-methyl-anthranilic acid

3-hydroxy-N-methyl-anthranilic acid [6] (138mg, 0.83mmol) was dissolved in HOAc (6ml) and flushed with argon. Bromine (85µl, 1.65mmol) was added and the reaction left over night. After evaporation of the solvent and co-evaporation with HOAc, HOAc (5ml) was added. Then the reaction mixture was flushed with argon and Br₂ (50µl, 0.97mmol) was added and the reaction was left at room temperature for 24 hours. HBr was azeotropically removed through evaporation four times with HOAc and twice with toluene. The residue was dissolved in MeOH (1.5ml), filtered and H₂O (1.5ml) was added to precipitate the product. The filtrate was concentrated and more of the product precipitated giving a total yield of 80 mg of 5-bromo-3-hydroxy-N-methyl-anthranilic acid. ¹H NMR(DMSO-d₆) :δ 10.16(br,1H), 7.36(d,J=2,2Hz,1H), 6.99(d,J=2.3Hz,1H), 3.5(br,2H), 2.93(s,3H). ¹³C NMR(DMSO-d₆):δ 168.90, 150.03, 139.86, 123.81, 121.46, 117.93, 109.05, 33.43. MS(TSP):m/z(rel.int.) 248/246(M⁺¹, 1.0/0.98)

### 4,5-Dibromo-3-hydroxy-N-methyl-anthranilic acid

5-Bromo-3-hydroxy-N-methyl-anthranilic acid (43mg, 0.17mmol) was mixed with HOAc (3ml) and flushed with argon. 48% HBr(aq) (600µl, 5.3mmol) and bromine (18µl, 0.35mmoL) was added and the reaction was heated to 70°C for 14 hours, protected from light. More bromine (5µl, 0.10mmol) was added and the reaction mixture was heated for another 18 hours. The mixture was evaporated and the residue was co-evaporated with HOAc and dried in vacuum. The product was purified by washing a solution of the crude product in 1M HCl with ether, followed by evaporation of the aqueous phase, yielding 4,5-dibromo-3-hydroxy-N-methyl-anthranilic acid (16mg). ¹H NMR(CD₃OD):δ8.07(s,1H), 4.97(br,3H), 3.15(s,3H). MS(TSP):m/z(rel.int.) 328/326/324(M⁺¹,0.53/1.0/0.48)

### Example 10 (Method E)

### 4,5-Dibromo-3-hydroxy-N,N-dimethylanthranilic acid

4,5-Dibromo-3-hydroxyanthranilic acid hydrochloride (100mg, 0.29mmol) was dissolved in acetonitrile (10ml), containing formaldehyde (115µl, 37% aqueous solution, 1.44mmol). NaCNBH₃ (55mg, 0.87mmol) was added and the reaction was left for 0.5 hour at room temperature, followed by addition of HCl (4M in MeOH) and evaporation. The residue was co-evaporated with MeOH, HOAc and MeOH. The crude product was purified by column chromatography (SiO₂, CH₃Cl-MeoH-HoAc 100:40:1) giving 4,5-dibromo-3-hydroxy-N,N-dimethylanthranilic acid (80mg). ¹H NMR(DMSO-d₆) :δ 8.83(br,1H), 7.48(s,1H), 2.92(s,6H). ¹³C NMR(DMSO-d₆) :δ 172.58, 166.78, 156.62, 135.77, 127.83, 123.58, 120.82, 41.09. MS(TSP):m/z(rel.int.) 342/340/338(M⁺, 0.51/1.0/0.53)

References
1. Todd W.P., Carpenter B.K. and Schwarcz R. Preparative Bio.Chem. 19, 155-65 (1989)
2. Brown P.E. and DeVries T. J.Am.Chem.Soc. 73, 1811-3 (1951)
3. Cox M.C.L., Diamont E.M. and Levy P.R. Recqeil 87, 1174-8 (1968)
4. Commercial available
5. Prudhomme M., Dauphin G., Guyot J., Jerminet G. J.Antibiotics 37, 627-34 (1984)
6. Keller O. Archiv der Pharmazie 246, 15-9 (1908)

### Pharmacological method

### Materials

[carboxy-¹⁴C]3-Hydroxyanthranilic acid (6 mCi/mmol) was received from Drs. E. Shaskan and L. Spitznagle (University of Connecticut, Farmington, CT, U.S.A.). [³H]QUIN was obtained from the Nuclear Research Center (Negev, Israel). All other chemicals and reagents were obtained from commercial suppliers.

### Tissue preparations

For routine assays, male Sprague-Dawley rats (150-200g) were killed by decapitation and their brains rapidly dissected onto ice. Whole forebrains or individual CNS regions were sonicated in four volumes (wt/vol) of distilled water, centrifuged at 50,000g for 20 min at 4°C, and the resulting supernatant used for the assay. For subcellular fractionation, the method of Mena et al. (1980) was used and the following fractions were collected: P₁ (nuclear fraction), P₂ (crude synoptosomal fraction), P₃ (microsomal fraction), soluble (cytosol fraction), myelin, synaptosomes, and mitochondria. All nonsoluble fractions were sonicated prior to assay.

### Measurement of 3HAO activity

For routine assays, 20µl of tissue extract (equivalent to 5 mg of original tissue wet weight) were incubated in the presence or absence of inhibitor (in 10 µl) at 37°C for 30 min in a solution containing 0.3 mM Fe (SO₄)₂, 38 mM 4-(2-hydroxyethyl)piperazine-1-ethanesulfonic acid (HEPES)/NaOH buffer (pH 6.0), and 5 µM ([¹⁴C]3HANA in a total volume of 195 µl. Blank values were obtained under identical conditions using tissue that had been heated for 5 min in a boiling water bath. The incubation was terminated by the addition of 50 µl 6% HClO₄, the tubes cooled on ice, and the precipitate removed by a 2-min centrifugation in a Beckman microfuge. 220 µl of supernatant were applied to a Dowex 50W (200-400 mesh) cation-exchange column (0.5 x 2 cm), which was washed with 1 ml of distilled H₂O to collect the [¹⁴C]QUIN produced. 5.5 ml of scintillation fluid were added to the eluate and its radioactivity determined by liquid scintillation spectrometry. Preliminary experiments had indicated that 90-95% of [¹⁴C]QUIN was collected by this procedure, whereas unreacted [¹⁴C]3HANA remained on the column.

### Pharmaceutical formulations

The administration in the novel method of treatment of this invention may conviently be oral, rectal, or parenteral at a dosage level of, for example, about 1 to 3000 mg/kg, preferably about 10 to 1000 mg/kg and especially about 25 to 250 mg/kg and may be administered on a regimen of 1 to 4 hours per day. The dose will depend on the route of administration, a particularly preferred route being by intravenous infusion of an aqueous solution containing a compound according to formula I. It will be appreciated that the severity of the disease, the age of the patient and other factors normally considered by the attending physician will influence the individual regimen and dosage most appropriate for a particular patient.

The pharmaceutical formulations comprising the compound of this invention may conveniently be tablets, pills, capsules, syrups, powders or granules for oral administration; sterile parenteral solutions or suspensions for parenteral administration; or as suppositories for rectal administration.

To produce pharmaceutical formulations containing a compound according to the present invention in the form of dosage units for oral application, e.g. lactose, saccharose, sorbitol, mannitol, starches such as potato starch, corn starch or amylopectin, cellulose derivatives, a binder such as gelatine or polyvinylpyrrolidone, and a lubricant such as magnesium stearate, calcium stearate, polyethylene glycol, waxes, paraffin, and the like, and then compressed into tablets. If coated tablets are required, the cores, prepared as described above, may be coated e.g. gum arabic, gelatine, talcum, titanium dioxide, and the like. Alternatively, the tablet can be coated with a polymer known to the man skilled in the art, dissolved in a readily volatile organic solvent or mixture of organic solvents. Dyestuffs may be added to these coatings in order to readily distinguish between tablets containing different active substances or different amounts of the active compounds.

For the preparation of soft gelatine capsules, the active substance may be admixed with e.g. a vegetable oil or polyethylene glycol. Hard gelatine capsules may contain granules of the active substance using either the above-mentioned excipients for tablets e.g. lactose, saccharose, sorbitol, mannitol, starches (e.g. potato starch, corn starch or amylopectin), cellulose derivatives or gelatine. Also liquids or semisolids of the drug can be filled into hard gelatine capsules.

Dosage units for rectal application can be solutions or suspensions or can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions, for example solutions containing from 0.2% to about 20% by weight of the active substance herein described, the balance being sugar and mixture of ethanol, water, glycerol and propylene glycol. Optionally such liquid preparations may contain colouring agents, flavouring agents, saccharine and carboxymethylcellulose as a thickening agent or other excipients known to the man in the art.

Solutions for parenteral applications by injection can be prepared in an aqueous solution of a water-soluble pharmaceutically acceptable salt of the active substance, preferably in a concentration of from about 0.5% to about 10% by weight. These solutions may also contain stabilizing agents and/or buffering agents and may conveniently be provided in various dosage unit ampoules.

## Claims

1. A compound of the general formula I 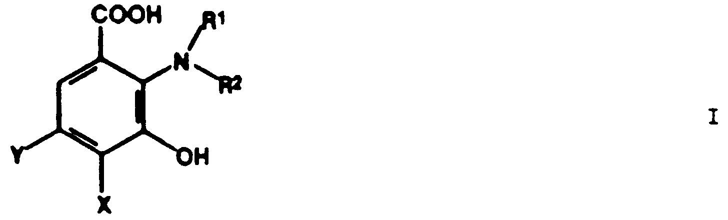 wherein
R¹ and R² are the same or different and selected from H and alkyl; and
X and Y are the same or different and selected from alkoxy, alkyl, alkylthio, fluoroalkyl, halogen, hydroxy, nitrile, OCF₃ and SCF₃,
or a pharmaceutically acceptable salt thereof.

2. A compound according to claim 1 wherein R¹ and/or R² representing an alkyl is a straight or branched lower alkyl.

3. A compound according to claim 2 wherein said alkyl is a C₁-C₆ alkyl.

4. A compound according to claim 1 wherein X and/or Y representing an alkyl is a straight or branched lower alkyl.

5. A compound according to claim 4 wherein said alkyl is a C₁-C₆ alkyl.

6. A compound according to claim 1 wherein X and/or Y representing an alkoxy is a lower alkoxy.

7. A compound according to claim 6 wherein said alkoxy is a C₁-C₆ alkoxy.

8. A compound according to claim 1 wherein X and/or Y representing a halogen is selected from iodo, flouro, chloro and bromo.

9. A compound according to claim 1 consisting of
5-bromo-4-chloro-3-hydroxyanthranilic acid,
4,5-dichloro-3-hydroxyanthranilic acid,
4-bromo-5-ethoxy-3-hydroxyanthranilic acid,
4,5-dibromo-3-hydroxyanthranilic acid,
4-bromo-3-hydroxy-5-methylanthranilic acid,
4,5-dibromo-3-hydroxy-5-methyl-anthranilic acid, and
a pharmaceutically acceptable salt thereof.

10. A process for the preparation of the compound of formula I according to claim 1, by
A) in case R¹ and R² are the same or different and selected from H and alkyl, Y is selected from Cl, Br and I, X is selected from alkoxy, alkyl, alkylthio, fluoralkyl, halogen, nitrile, OCF₃ and SCF_{3;}
halogenating a compound of formula II 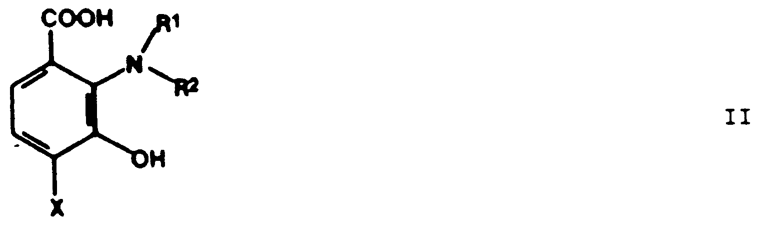 wherein R¹, R² and X are as defined in A) above, or
B) in case R¹ and R² are H, X and Y are the same or different and selected from alkoxy, alkyl, alkylthio, fluoroalkyl, halogen, hydroxy, nitrile, OCF₃ and SCF₃;
deprotecting a compound of formula III 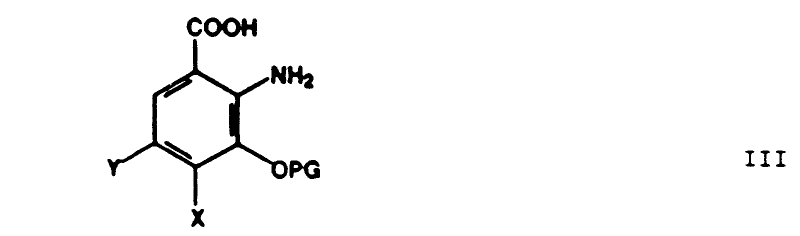 wherein R¹, R², X and Y are as defined in B) above and PG is a protecting group, or
C) in case R¹ and R² are H, X and Y are the same or different and selected from alkoxy, alkyl, alkylthio, fluoroalkyl, halogen, hydroxy, nitrile, OCF₃ and SCF₃;
reducing a compound of formula IV 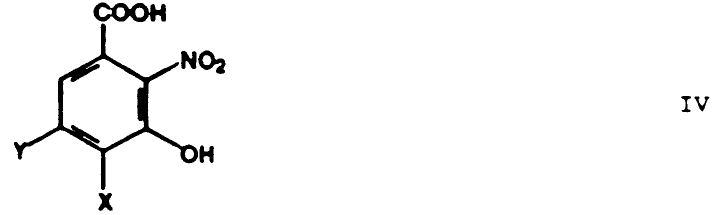 wherein X and Y are as defined in C) above, or
D) in case R¹ and R² are the same or different and selected from H and alkyl, X is Cl, Br or I and Y is selected from alkoxy, alkyl, fluoroalkyl and halogen;
halogenating a compound of formula V 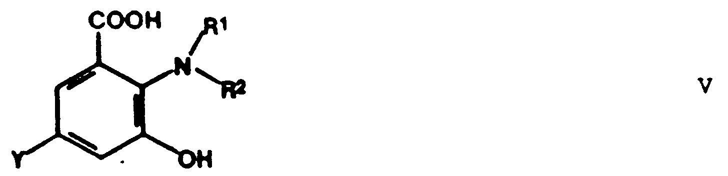 wherein R¹, R², and Y are as defined in D) above, or
E) in case R¹ is alkyl, R² is H or alkyl and X and Y are selected from alkoxy, alkyl, alkylthio, fluoroalkyl, halogen, hydroxy, nitrile, OCF₃ and SCF_{3;}
alkylating a compound of formula VI 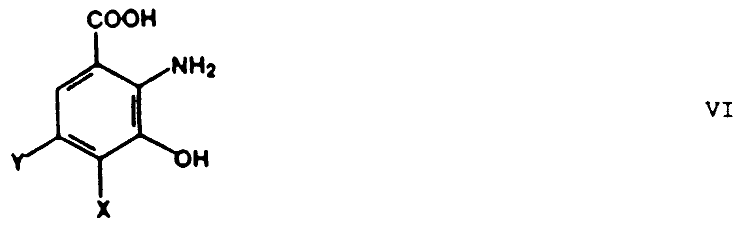 wherein X and Y are as defined in E) above, or
F) in case R¹ and R² are the same or different and selected from H and alkyl, X and Y are the same or different and selected from alkoxy, alkyl, alkylthio, fluoroalkyl, halogen, hydroxy, nitrile, OCF₃ and SCF₃;
deesterifying a compound of formula VII 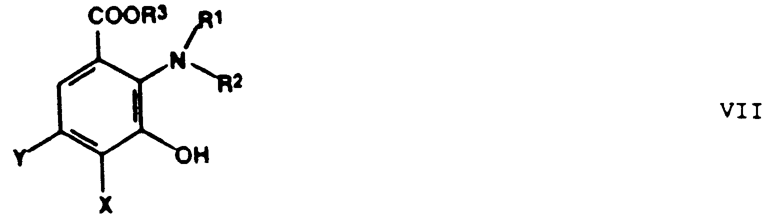 wherein R¹, R², X and Y are as defined in F) above and R³ is selected from alkyl, benzyl, 1,1,1-trichloroethyl and 2-(trimethylsilyl)ethoxymethyl (SEM).

11. A pharmaceutical formulation containing a compound according to claim 1 as active ingredient and a pharmaceutically acceptable carrier.

12. A compound according to claim 1 for use in therapy.

13. A compound as defined in claim 12 for use as an agent for prevention or treatment of neurodegeneration.

14. The use of a compound according to claim 1 for the manufacture of a medicament for the prevention or treatment of neurodegeneration.

15. A method for the prevention or treatment of neurodegeneration by administrering to a host in need of such a treatment a sufficient amount of a compound according to claim 1.

16. A compound of the general formula II 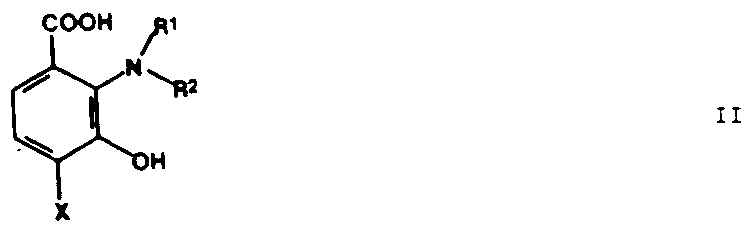 wherein R¹ and R² are the same or different and selected from H and alkyl, and X is selected from alkoxy, alkyl, alkylthio, fluoralkyl, halogen, nitrile, OCF₃ and SCF₃ with the proviso that the compounds of formula II wherein R¹ and R² are H and X is OMe, OEt, Cl, Br or F are excluded,

17. A compound of the general formula III 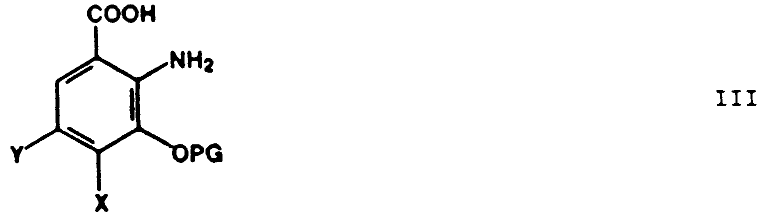 wherein X and Y are the same or different and selected from alkoxy, alkyl, alkylthio, fluoroalkyl, halogen, hydroxy, nitrile, OCF₃ and SCF₃ and PG is a protecting group, with the proviso that the compounds of formula III wherein PG is Me, X is OMe and Y is OMe; PG is Me, and X and Y are Cl; and PG is Me, X is OMe and Y is Cl are excluded.

18. A compound of the general formula IV 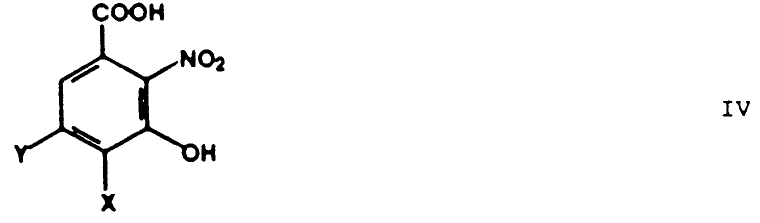 wherein X and Y are the same or different and selected from alkoxy, alkyl, alkylthio, fluoroalkyl, halogen, hydroxy, nitrile, OCF₃ and SCF_{3.}

19. A compound of the general formula V 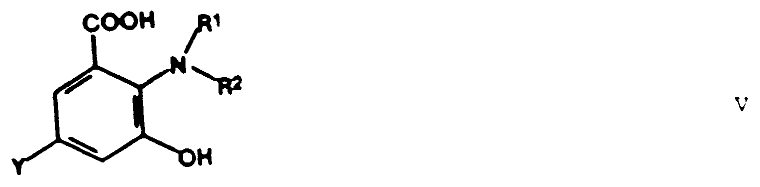 wherein R¹ and R² are the same or different and selected from H and alkyl, and Y is selected from alkoxy, alkyl, fluoroalkyl, halogen, nitrile, OCF₃ and SCF_{3.}

20. A compound of the general formula VI 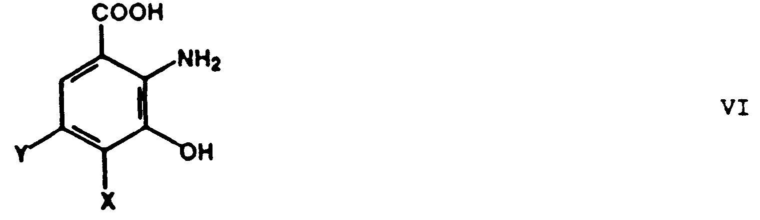 wherein X and Y are selected from alkoxy, alkyl, alkylthio, fluoroalkyl, halogen, hydroxy, nitrile, OCF₃ and SCF_{3.}

21. A compound of the general formula VII 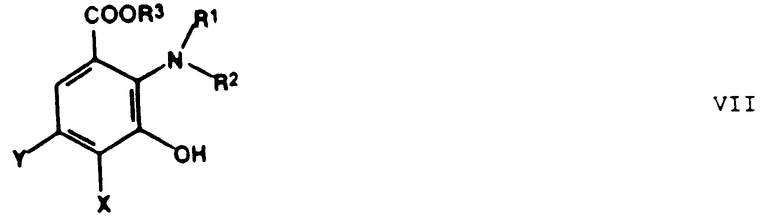 wherein R¹ and R² are the same or different and selected from H and alkyl, X and Y are the same or different and selected from alkoxy, alkyl, alkylthio, fluoroalkyl, halogen, hydrogen, nitrile, OCF₃ and SCF₃ and R³ is selected from alkyl, benzyl, 1,1,1-trichloroethyl and 2-(trimethylsilyl)ethoxymethyl (SEM).
